# EUROPEAN PATENT SPECIFICATION

(11) **EP 1 129 719 B1**
(45) Date of publication and mention of the grant of the patent: **08.06.2005**
(21) Application number: 00103598.9
(22) Date of filing: 21.02.2000
(51) Int. Cl.: A61K 38/18

(54) **Treatment of psoriasis through down-regulation of the EGF-receptor with topically-applied EGF**
Topische Behandlung von Psoriasis mit EGF zur Herbabsetzungsregulierung der EGFR
Traitement du psoriasis par régulation négative du récepteur de l'EGF avec EGF, appliqué localement

(43) Date of publication of application: 05.09.2001
(73) Proprietor: Neirinckx, Rudi Dominique, Dr., 68440 Dietwiller (FR)
(72) Inventor: Neirinckx, Rudi Dominique, Dr., 68440 Dietwiller (FR)
(74) Representative: Bohmann, Armin K., Dr.

(56) References cited:
- NANNEY L B ET AL: "MODULATION OF EPIDERMAL GROWTH FACTOR RECEPTORS IN PSORIATIC LESIONS DURING TREATMENT WITH TOPICAL EGF" JOURNAL OF INVESTIGATIVE DERMATOLOGY, vol. 98, no. 3, 1992, pages 296-301, XP000946863 ISSN: 0022-202X
- CASACO A ET AL: "Topical anti-inflammatory activity of human recombinant epidermal growth factor." SKIN PHARMACOLOGY AND APPLIED SKIN PHYSIOLOGY, vol. 12, no. 1-2, January 1999 (1999-01), pages 79-84, XP000946866 ISSN: 1422-2868
- BROWN G L ET AL: "ENHANCEMENT OF WOUND HEALING BY TOPICAL TREATMENT WITH EPIDERMAL GROWTH FACTOR" NEW ENGLAND JOURNAL OF MEDICINE, vol. 321, no. 2, 1989, pages 76-79, XP000946887 ISSN: 0028-4793

## Description

### INTRODUCTION

Psoriasis is a chronic skin disorder that affects approximately 4.0 million people in the US., and annual treatment costs in the USA alone are estimated at over $1.5 billion. There are no currently available drugs for this disease that offer satisfactory efficacy and safety. Psoriatic lesions are caused by the hyperproliferation of keratinocytes, but it has been demonstrated that EGF-R signalling is required for the growth of keratinocytes.

It has been demonstrated that the upper epidermal layer in psoriatic tissue contains levels of EGF-receptors (EGFR) more similar to the levels found in the mitotically-active basal cell layer of skin. In normal epidermis r-EGF is located primarily in the germinative layer, which contains r-EGF levels 4 times higher than those found in the more-diferentiated cells of the upper epidermal layers. In psoriatic lesions the upper epidermal layers shows r-EGF levels 2x higher than in normal tissue, while the germanitive layer has normal levels.( L.A. Nanney et al; J. Invest.Dermat. Vol 85, p 260-265).

There is only a poor correlation between the levels of r-EGF and the level of cellular proliferation. An example of cells with elevated metabolism but low mitotic activity is the case of the sweat duct epithelium. Similarly, the high level of r-EGF indicates elevated metabolism rather than lack of differentiation in psoriatic lesions.

### PROPOSAL

As the main difference in r-EGF distribution in normal and psoriatic tissue is the abnormal retention of the receptor beyond the first 2-3 cell rows in the stratum basilis in psoriatic tissue , we propose to reduce these concentrations through a down regulation of the receptor using higher than normal levels of EGF at the level of the receptor.

This is similar to the down regulation of FSH and LH excretion through the saturation of pituitary GnRH receptors in response to a constant level of GnRH.

This down regulation is due to the deviation from the normal physiological situation where intermittent surges of GnRH release LH and FSH ,without causing saturation of the receptors. It is also similar to the effect of high levels of estradiol on estrogen-dependent tumour lines: In-vitro , the proliferation of these cells can be halted by high, non-physiological concentrations of the hormone.

It has been reported that high levels of EGF have inhibited the growth of EGF-dependent cancer cell lines in-vitro. The biological activity of epidermal growth factor (EGF) is mediated through the intrinsic tyrosine kinase activity of the EGF receptor (EGFR). In numerous cell types, binding of EGF to the EGFR stimulates the tyrosine kinase activity of the receptor eventually leading to cell proliferation. In tumor-derived cell lines, which overexpress the EGFR, however, growth inhibition is often seen in response to EGF. The mechanism for growth inhibition is unclear. A constant pressure of EGF may engender a similar down regulation of the EGF receptors and result in a more-normal metabolic activity and a reversion of psoriatic tissue to normal.

### CLINICAL EVALUATION

Two patients, suffering from psoriasis , were treated with a topical cream containing sulfadiazine and 5 µg of EGF/gram of cream. The treatment was carried out by applying 2 grams of cream over each psoriatic lesion and was carried out twice a day for a week.

### RESULTS AND CONCLUSION

After a week's treatment the psoriatic lesions showed - subjectively - a marked improvement, comparable to the result obtained after treatment with corticosteroids.
It is therefore felt that a larger clinical trial is warranted.

### INVENTION

The present invention is directed to the use of epidermal growth factor (EGF) for the manufacture of a medicament for the treatment of psoriasis, whereby the medicament is for topical treatment and provides non-physiologic high concentrations of EGF which can down-regulate the expression of the EGF-receptor.

Further embodiments of the invention are specified in the claims.

## Claims

1. Use of epidermal growth factor (EGF) for the manufacture of a medicament for the treatment of psoriasis, whereby the medicament is for topical treatment and provides non-physiologic high concentrations of EGF which can down-regulate the expression of the EGF-receptor.

2. Use according to claim 1, wherein the medicament is a formulation for topical treatment.

3. Use according to claim 2, wherein the formulation contains 0.1 to 50 micrograms EGF/gram of formulation.

4. Use according to claim 3, wherein the formulation contains 5 - 20 micrograms EGF/gram of formulation.

5. Use according to claim 4, wherein the formulation is a cream and contains 0.1-3% sulfadiazine.

6. Use according to claim 5, wherein the formulation contains 10 µg EGF/gram of formulation, and 1% sulfadiazine.

7. Use according to claim 1 or 2, wherein the formulation contains anti-inflammatory products.

8. Use according to claim 7, wherein the formulation contains other dermatologically beneficial products.

## Patentansprüche

1. Verwendung von epidermalem Wachstumsfaktor (EGF) für die Herstellung eines Medikaments für die Behandlung von Psoriasis, wobei das Medikament für die topische Behandlung ist und nicht-physiologische hohe Konzentrationen an EGF bereitstellt, die die Expression des EGF-Rezeptors herunterregeln können.

2. Verwendung nach Anspruch 1, wobei das Medikament eine Formulierung für die topische Behandlung ist.

3. Verwendung nach Anspruch 2, wobei die Formulierung 0,1 - 50 µg EGF/g Formulierung enthält.

4. Verwendung nach Anspruch 3, wobei die Formulierung 5 - 20 µg EGF/g Formulierung enthält.

5. Verwendung nach Anspruch 4, wobei die Formulierung eine Creme ist und 0,1 - 3 % Sulfadiazin enthält.

6. Verwendung nach Anspruch 5, wobei die Formulierung 10 µg EGF/g Formulierung und 1 % Sulfadiazin enthält.

7. Verwendung nach Anspruch 1 oder 2, wobei die Formulierung anti-entzündliche Produkte enthält.

8. Verwendung nach Anspruch 7, wobei die Formulierung andere dermatologisch vorteilhafte Produkte enthält.

## Revendications

1. Utilisation du facteur de croissance de l'épiderme (EGF) pour la fabrication d'un médicament pour le traitement du psoriasis, le médicament étant destiné au traitement topique et fournissant des concentrations élevées d'EGF non-physiologiques qui peuvent réguler vers le bas l'expression du récepteur à l'EGF.

2. Utilisation selon la revendication 1, dans laquelle le médicament est une formulation pour traitement topique.

3. Utilisation selon la revendication 2, dans laquelle la formulation contient de 0,1 à 50 microgrammes d'EGF par gramme de formulation.

4. Utilisation selon la revendication 3, dans laquelle la formulation contient de 5 à 20 microgrammes d'EGF par gramme de formulation.

5. Utilisation selon la revendication 4, dans laquelle la formulation est une crème et contient 0,1 à 3 % de sulfadiazine.

6. Utilisation selon la revendication 5, dans laquelle la formulation contient 10 µg d'EGF par gramme de formulation, et 1 % de sulfadiazine.

7. Utilisation selon la revendication 1 ou 2, dans laquelle la formulation contient des produits anti-inflammatoires.

8. Utilisation selon la revendication 7, dans laquelle la formulation contient d'autres produits bénéfiques sur le plan dermatologique.
